# EUROPEAN PATENT SPECIFICATION

(11) **EP 1 211 988 B1**
(45) Date of publication and mention of the grant of the patent: **17.02.2010**
(21) Application number: 00950189.1
(22) Date of filing: 01.08.2000
(51) Int. Cl.: A61B 17/12, A61F 2/48, A61F 5/00

(54) **STOMA OPENING FORMING APPARATUS**
GERÄT ZUR FORMUNG EINER STOMAÖFFNUNG
DISPOSITIF SERVANT A FORMER UN ORIFICE DE STOMIE

(30) Priority: 12.08.1999 US 373224
(43) Date of publication of application: 12.06.2002
(73) Proprietor: Potencia Medical AG, 6341 Baar (CH)
(72) Inventor: FORSELL, Peter, CH-6313 Menzingen (CH)
(74) Representative: Mercer, Christopher Paul
(86) International application number: PCT/SE2000/001531
(87) International publication number: WO 2001/012078

(56) References cited:
- WO-A1-00/09048
- WO-A1-00/15158
- WO-A1-99/18885
- US-A- 4 908 011
- US-A- 5 771 903
- US-A- 5 938 669

## Description

The present invention relates to an apparatus for forming a stoma opening in the stomach or esophagus of a patient. The apparatus comprises an implantable elongated restriction member adapted to be formed into a substantially closed loop around the patient's stomach or esophagus, so that the loop defines a restriction opening.

This kind of apparatus in the form of a gastric banding device, in which a band encircles a portion of a patient's stomach to restrict the food intake of the patient, have been used in surgery for morbid obesity to form a small gastric pouch above the band and a reduced stoma opening in the stomach. Although such a band is applied around the stomach to obtain an optimal stoma opening during surgery, some prior gastric banding devices are provided with an adjustment means enabling a minor post-operation adjustment of the size of the stoma opening. In all such prior art devices, such as disclosed in U.S. Patent No. 4 592 339, European Patent No. 0611561 and International Patent Application WO 94/27504, the adjustment means typically comprises an inflatable cavity in the band and an injection port in fluid connection with the inflatable cavity. The injection port is subcutaneously implanted to allow the addition of fluid to or withdrawal of fluid from the cavity by an injection needle penetrating the patient's skin and passing into the injection port. In practice, the band is made of silicone which is a material approved for implantation and the fluid is a liquid such as an isotonic salt solution mixed with other conventional materials.

Thus, the only way for a patient carrying a gastric band of the type used in the above-discussed prior art devices to have the stoma opening adjusted is by visiting a doctor or nurse who is able to use an injection needle for withdrawing some liquid from or adding some liquid to the band. However, a problem with the prior art devices is that when the patient lies in bed sleeping it may happen that the stoma opening is completely closed by the stomach wall, which might cause the patient to vomit and feel sick. In such a situation the patient normally has no doctor or nurse available.

The kind of apparatus presented initially has also been used for treating heartburn and reflux disease due to hiatal hernia, *i*.*e*. a portion of the stomach immediately below the gastric fundus slides upwardly through the esophageal hiatus. In consequence, stomach acids and foods are regurgitated into the esophagus. In the late 1970s a prior art prosthesis called Angelchik, according to U.S. Patent No. 3 875 928, was used to operatively treat heartburn and reflux disease. However, the Angelchik prosthesis had a major disadvantage in that it was not possible to adjust the size of the restriction opening after the operation. A further disadvantage was that the prosthesis did not satisfactorily protect the esophagus and the surrounding area against injuries due to poor shape of the prosthesis. Therefore, operations using the Angelchik prosthesis are no longer practised.

An operation technique, semi-fundoduplicatio, is currently in use for treating heartburn and reflux disease. A most common operation is Nissen semi-fundoduplicatio, in which one takes the fundus of the stomach and makes a three quarter of a turn around the esophagus and sutures between the stomach and esophagus. Although this operation works fairly well it has three main disadvantages. Firstly, most patients treated in accordance to semi-fundoduplicatio lose their ability to belch. Secondly, many of these patients get dysphagia, *i*.*e*. difficulties to swallow after the operation. Thirdly, it is not possible to adjust the stoma opening in the esophagus or stomach in any way after the operation. Characteristic for these patients is the variation of their problems over the day. For example, many patients have difficulties during the night when they lie down because of stomach acid leaking up into the esophagus.

US 5,938,669 discloses an adjustable gastric banding device used for contracting a patient's stomach in order to fight obesity in which a gastric band including a cavity filled with liquid is implanted around the stomach. The cavity is connected by a tube to a control box and a balancing reservoir which are implanted under the patient's skin. The box contains an electric pump and an elecronic control unit capable of communicating by radio with a monitor carried by the patient and with a controller intended for the doctor.

The prime object of the present invention is to provide a new apparatus for forming a stoma opening in the stomach or esophagus of a patient, wherein the new apparatus is suited for treating obese patients as well as patients suffering from heartburn and reflux disease.

Another object of the present invention is to provide a new convenient apparatus for forming a stoma opening, which practically eliminates the patient's need for visiting a doctor or nurse in order to adjust the stoma opening so that the patient always is satisfied.
Accordingly, the present invention provides:
an apparatus for forming a stoma opening in the stomach or esophagus of a patient, the apparatus comprising: an implantable elongated restriction member adapted to be formed into a substantially closed loop around the patient's stomach or esophagus, so that the loop defines a restriction opening, an implantable adjustment device for adjusting the restriction member in the loop to change the size of the restriction opening, a control device for controlling the adjustment device to adjust the restriction member to change the size of the restriction opening, and an implantable sensor for sensing at least one physical parameter associated with the patient, wherein the control device is adapted to control the adjustment device in response to the sensor sensing a change in the physical parameter. Hereby the patient's stoma opening will be similarly adjusted when the elongated restriction member is implanted in the patient.

The at least one physical parameter associated with the patient may be the pressure in the patient's stomach or esophagus, or the orientation of the patient with respect to the horizontal, wherein the control device is adapted to control the adjustment device in response to the sensor sensing a change in the physical parameter.

The control device may comprise an implantable internal control unit for directly controlling the adjustment device in response to signals from the sensor. Alternatively, the control device may comprise an external control unit outside the patient's body for directly or indirectly controlling the adjustment device in response to signals from the sensor. The external control unit may store information on the physical parameter sensed by the sensor and be manually operated to control the adjustment device based on the stored information.

Conveniently, the apparatus may further comprise at least one implantable sender for sending information on the physical parameter sensed by the sensor.

In accordance with a particular embodiment of the invention, the sensor comprises a pressure sensor for sensing as the physical parameter the pressure in the patient's stomach or esophagus. The pressure sensor may be any suitable known or conventional pressure sensor such as shown in U.S. patents 5 540 731, 4 846 181, 4 738 267, 4 571 749, 4 407 296 or 3 939 823; or an NPC-102 Medical Angioplasty Sensor.

Preferably, the control device is adapted to control the adjustment device to change the size of the restriction opening in response to the pressure sensor sensing a change in the pressure in the stomach or esophagus.

In the case of treating obese patients, in accordance with one embodiment of the invention the control device controls the adjustment device to reduce the restriction opening in response to the pressure sensor sensing a pressure, within a normal pressure range, equal to or exceeding a predetermined value, and to enlarge the restriction opening in response to the pressure sensor sensing a pressure below a predetermined value. The predetermined value is the pressure that normally occurs preferably in the stomach or, alternatively, in the esophagus soon after the patient has started to eat. Thus, food reaching the stomach causes an increase in the pressure in the stomach.

Between meals, for example at night, when the stomach is empty and the sensed pressure is well below the predetermined high pressure, the adjustment device can keep the restriction opening as large as possible, *i*.*e*. substantially fully open. As a result, the stoma opening will be relatively large, which minimizes the risk of the stoma opening closing completely. When the obese patient eats, so that the food entering the stomach increases the pressure therein, the restriction opening is reduced when the pressure sensor senses the predetermined value. Consequently, the obese patient's hunger is soon satisfied after a relatively small intake of food, which will lead to a reduction of the obese patient's weight.

In the case of treating patients suffering from heartburn and reflux disease, in accordance with another embodiment of the invention, the control device controls the adjustment device to enlarge the restriction opening in response to the pressure sensor sensing a pressure, within a normal pressure range, equal to or exceeding a predetermined value, and to reduce or close the restriction opening in response to the pressure sensor sensing a pressure below a predetermined value. The predetermined value is the pressure that occurs preferably in the esophagus or, alternatively, in the stomach soon after the patient has started to eat. Thus, food reaching the esophagus (or stomach) causes an increase in the pressure in the esophagus (or stomach).

Between meals, for example at night, when the stomach is empty and the sensed pressure is well below the predetermined value, the control device controls the adjustment device to adjust the restriction device to close the stoma opening. Consequently, the restriction device will work as an artificial sphincter.

In addition to or as an alternative to the two embodiments described above related to the treatment of obese patients and patients suffering from heartburn and reflux disease in which normal pressures in the stomach or esophagus are sensed, the control device may be adapted to control the adjustment device to enlarge the restriction opening in response to the pressure sensor sensing a pressure equal to or exceeding an excessively high value which is injurious to the patient. For example, an injurious pressure can occur in the stomach or esophagus if a large piece of food get stuck in the stoma opening. By enlarging the restriction opening the piece of food will be able to pass through the correspondingly enlarged stoma opening.

Conveniently, the pressure sensor may indirectly sense the pressure in the stomach by sensing the pressure exerted by the stomach or esophagus against the restriction member.

In accordance with another particular embodiment of the invention, the sensor comprises a position sensor for sensing as the physical parameter the orientation of the patient with respect to the horizontal. The position sensor may be a biocompatible version of what is shown in U.S. patents 4 942 668 and 5 900 909.

In the case of treating an obese patient, the control device controls the adjustment device to increase the restriction opening in response to the position sensor sensing that the patient has assumed a substantially horizontal orientation, *i*.*e*. that the patient is lying.

In the case of treating a patient suffering from heartburn and reflux disease, the control device controls the adjustment device to reduce or close the restriction opening in response to the position sensor sensing that the patient has assumed a substantially horizontal orientation, *i*.*e*. that the patient is lying.

Alternatively, the apparatus may further comprise such a position sensor in addition to the above described pressure sensor.

The control device may control the adjustment device in response to the time of day and for that purpose it suitably comprises a clock mechanism used for controlling the adjustment device to adjust the restriction member to keep the restriction opening at different sizes during different time periods of the day. In case a sensor of any of the above described types (pressure or position sensor) is provided, the clock mechanism is used for controlling the adjustment device provided that the physical parameter sensed by the sensor does not override the clock mechanism.

Preferably, the control device comprises an internal control unit to be implanted in the patient and a wireless remote control adapted to set control parameters of the internal control unit from outside the patient without mechanically penetrating the patient, when the internal control unit is implanted in the patient. At least one of the control parameters, which is settable by the wireless remote control, is the physical parameter. Suitably, the wireless remote control may set the above mentioned clock mechanism.

For example the wireless remote control may comprise a signal (*e*.*g*. electromagnetic or sound waves, magnetic energy, digital pulses, etc.) transmitter, an implantable signal receiver, and an implantable energizer unit for transforming wireless energy from the signals, as they are transmitted from the transmitter to the signal receiver, into energy different than the wireless energy for energizing the adjustment device and/or the sensor. Alternatively, the apparatus may comprise an implantable battery or accumulator, such as a capacitor, for energizing the adjustment device and/or the sensor.

The adjustment device may comprise an expandable cavity in the restriction member, the size of the restriction opening being reduced upon expansion of the cavity and increased upon contraction of the cavity, and a reservoir for hydraulic fluid (*e*.*g*. a salt solution). In this case the adjustment device is adapted to distribute hydraulic fluid from the reservoir to expand the cavity, and to distribute hydraulic fluid from the cavity to the reservoir to contract the cavity, to thereby change the size of the restriction opening. The reservoir may be attached or fixed to the restriction member, or integrated therewith. Further, the adjustment device comprises a pump for pumping fluid between the cavity and the reservoir, and adapted to be subcutaneously implanted in the patient remote from the restriction member. Alternatively, the pump may be attached or fixed to the restriction member. In accordance with a preferred embodiment of the invention, the reservoir, pump and restriction member form a single piece, suitably together with the sensor.

Alternatively, the restriction member may be non-inflatable, which has the advantage that the risk of fluid leaking from the restriction member is avoided. In this case it is preferred to use an adjustment device which is designed to mechanically adjust the non-inflatable restriction member.

Suitably, an implantable battery or accumulator, such as a capacitor, may be provided for energizing the adjustment device and/or the sensor.

The invention is not limited to sensing the pressure in the patient's stomach or esophagus, or the patient's orientation with respect to the horizontal, but may sense a wide variety of other physical parameters associated with the patient, such as parameters associated with rest or sleep, etc.

By using the apparatus of the present invention for treating an obese patient it is possible to minimize or eliminate nausea in the patient. Thus, a sensor of the apparatus is surgically implanted in the patient, at least one physical parameter of the patient is sensed using the sensor, and if in response to the sensor it is determined that a significant change has occurred in the physical parameter the adjustment device is controlled to enlarge the restriction opening (*e*.*g*. open it substantially fully), so that nausea is minimized or substantially eliminated. Where the sensor is a pressure sensor, the restriction opening is substantially fully open when the pressure in the stomach is at a pressure value commonly occurring when the obese patient is sleeping at night, or other between meal times. Where the sensor is a position sensor, the adjustment device is controlled to enlarge the restriction opening if the obese patient is substantially horizontal.

An internal control unit of the control device may be implanted in the patient at substantially the same time as the sensor, so that the internal control unit is mounted on the restriction member, or at some other location associated with the implant. Suitably, the internal control unit is operated exteriorly of the patient in a non-invasive manner to control the adjustment device.

The present invention is described in more detail in the following with reference to the accompanying drawings, in which
FIGURE 1 is a schematic perspective view of the torso of an obese human having an apparatus for forming a stoma opening according to the invention, showing internal body portions of the human schematically for clarity of illustration;
FIGURE 2 is a schematic side view, with portions cut away for clarity of illustration, of an exemplary apparatus according to the invention used in the human body as illustrated in FIGURE 1; and
FIGURE 3 is a view like that of FIGURE 2 only of another exemplary embodiment according to the invention.

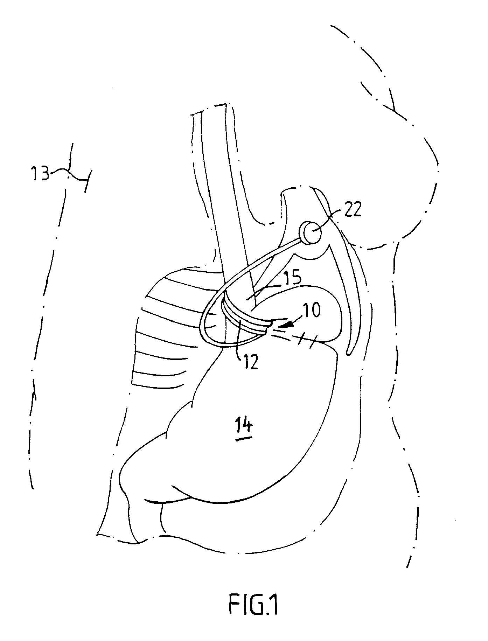

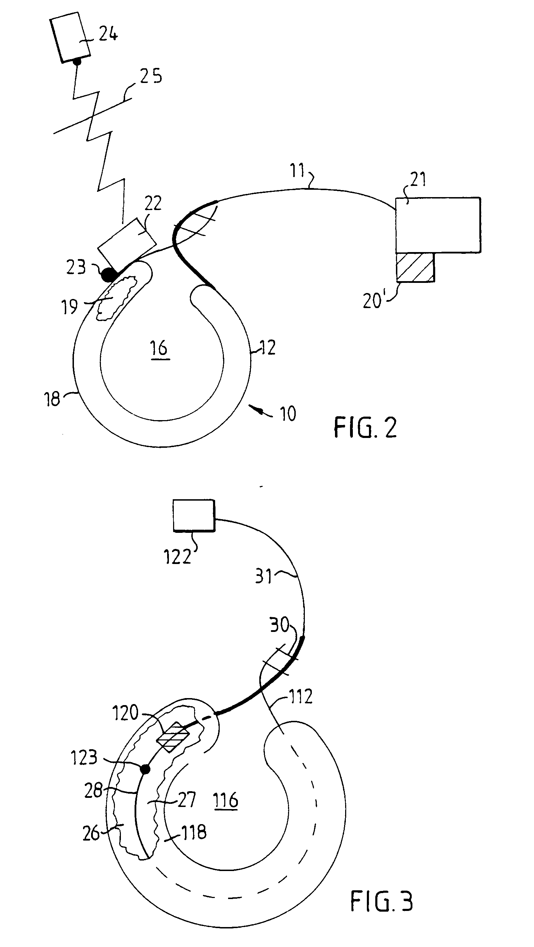

An apparatus according to the present invention for forming a stoma opening in the stomach (or esophagus) of an obese human patient is illustrated schematically at 10 in FIGURES 1 and 2. The apparatus 10 includes an elongated restriction member 12, such as a gastric band, which is surgically implanted in the human body 13 around the human's stomach 14 or -- as shown in the embodiment of FIGURE 1 - both the stomach and esophagus 15 to form a stoma opening between an upper small pouch of the stomach 14 and a lower major portion of the stomach 14. The elongated restriction member 12 is formed into a substantially closed loop, the loop defining a restriction opening, such as illustrated schematically at 16 in FIGURE 2 (FIGURE 2 shows the opening 16 dimensioned, compared to the illustration in FIGURE 1, in a manner whereby it would be disposed around the stomach 14 rather than the esophagus 15).

In the case of treating a human patient suffering from heartburn and reflux disease, the restriction member 12 would be applied around the esophagus or around an upper portion of the stomach close to the cardia without forming the upper pouch of the stomach illustrated in FIGURE 1.

The restriction member 10 includes an adjustment device, which may be of any suitable type. In the embodiment illustrated in FIGURE 2 the adjustment device comprises an expandable element 18 integrated with the band 12 and defining an interior cavity 19. An implanted pump 20 remote from the band 12 is connected thereto via a fluid conduit 11. The pump 20 is fixed to a reservoir, likewise implanted, for hydraulic fluid, shown generally at 21 in FIGURE 2. The hydraulic fluid would be any suitable substantially incompressible fluid, which will not cause severe illness or injury to the human if it were to leak from the reservoir 21 or the cavity 19, such as a salt solution. By pumping hydraulic fluid from the reservoir 21 through the conduit 11 into the cavity 19, the pump 20 causes the element 18 to expand thereby reducing the size of the opening 16, whereas by pumping hydraulic fluid out of the cavity 19 into the reservoir 21 the pump 20 causes the element 18 to contract, causing the opening 16 to enlarge.

The pump 20 may be controlled by a control unit 22. While the control unit 22 may be mounted exteriorly of the body 13, in the preferred embodiment the control unit 22 is mounted within the body 13, preferably on the elongated restriction member 12, and adjacent the cavity 19. Electrical interconnections (not shown) are provided between the control unit 22 and the pump 20. A battery for operating the control unit 22 and pump 20 may be provided right within the control unit 22. Alternatively, a power source for powering the control unit 22 and the pump 20 may be located exteriorly of the body 13. Energy from such an exterior power source may be wirelessly transmitted to implanted energy consuming components.

A pressure sensor 23, shown schematically in FIGURE 2, is implanted in the body 13 of the human patient for sensing the pressure in the stomach 14. For example, in the embodiment illustrated in FIGURE 2 the pressure sensor 23 is mounted on the restriction member 12 and indirectly senses the pressure in the stomach 14 by sensing the pressure exerted by the stomach against the expandable element 18. However, the sensor 23 may be mounted directly on the inner side of the elongated restriction member 12 at a location remote from the cavity 19 to directly abut the stomach, or any other suitable mounting may be provided as long as the pressure sensor 23 is able to sense the pressure or related value within stomach 14 that is caused when food is ingested by the human patient.

The sensor 23 may be any suitable known or conventional sensor which is capable of performing the functions as set forth above. Some non-limiting examples of implantable sensors include those described in U.S. patents 5 540 731, 4 846 181, 4 738 267, 4 571 749, 4 407 296 or 3 939 823, and the NPC-102 Medical Angioplasty Sensor.

Alternatively the adjustment device may mechanically adjust the restriction member 12, *e*.*g*. a motor may be provided to adjust member 12.

The control unit 22 may be of the type which communicates effectively with a wireless remote control 24 illustrated schematically in FIGURE 2, with the zig-zag line between the elements 22, 24 indicating wireless communication therebetween, and the solid cross line 25 indicating that the remote control 24 is exterior of the body 13. The remote control 24 may be for setting control parameters of the control unit 22 from outside the body 13 without mechanically penetrating the human patient. One of the control parameters which is settable by the device 24 may be the predetermined pressure values that the sensor 23 senses and communicates to the control unit 22 (either by electrical connections, or in a wireless manner) to cause the pump 20 to operate and hydraulic fluid to be removed from or pumped into the cavity 19. Wireless energy carrying signals from the remote control 24 may be electromagnetic or sound or other types of waves, magnetic transfer, or digital pulses.

In one embodiment of the invention the control unit 22 may include a clock mechanism mounted on the restriction member 12 and used for controlling the adjustment device 17 to adjust the restriction member 12 to keep the restriction opening 16 at different sizes during different time periods of the day, provided that the pressure sensed by the pressure sensor 23 does not exceed a predetermined value (which would indicate food in the stomach 14). For example in the middle of the night when it is expected that the obese human would have little or no food in his/her stomach 14, the control unit 22 would automatically operate the pump 20 to pump fluid out of the cavity 19 into the reservoir 21 so as to substantially fully open the restriction opening 16, whereas at other times of the day the pump 20 could be controlled to vary the size of the restriction opening 16 from the maximum to a minimum value.

The remote control 24 may be used to set the clock mechanism 22.

For example, the wireless remote control 24 may comprise a signal transmitter, and a signal receiver may be implanted within the body 13 (*e*.*g*. as part of the control unit 22), and an energizer unit may also be implanted in the body 13 (*e*.*g*. as part of the control unit 22) for transforming wireless energy from the signals as they are transmitted to the signal receiver into energy different than the wave energy, for example electrical energy, for energizing the adjustment device (*e*.*g*. by operating the pump 20) and sensor 23. Alternatively a battery may be implanted in the body 13 (*e*.*g*. as part of the control unit 22) for energizing the adjustment device (*e*.*g*. the pump 20) and sensor 23, or an accumulator (such as a capacitor) may be implanted in the patient for energizing the adjustment device and sensor 23.

The present invention can be used in a method for minimizing or eliminating nausea in an obese human having an apparatus 10, as a result of the stoma opening 16 substantially closing between meals. The method comprises: (a) Implanting (*e*.*g*. with a conventional surgical procedure) the adjustment device and pressure sensor 23 in the obese human's body 13 operatively associated with the restriction opening 16. (b) Sensing the pressure in the stomach 14; and (c) if in response to (b) it is determined that the pressure is below a predetermined value (indicating little or no food in the stomach 14), then controlling the adjustment device to substantially fully open the restriction opening 16, so that nausea is minimized or substantially eliminated. In the method (b) and (c) may be practised to substantially fully open the restriction opening 16 when the pressure in the stomach is at a pressure value commonly occurring when the human is sleeping at night.

The method may further comprise implanting the control unit 22 in the human's body 13 at substantially the same time that (a) is practised so that the control unit 22 is mounted on the restriction member 12 or at some other location associated with the implant, and operating the control unit 22 exteriorly of the human in a non-invasive manner (as by using the remote control 24) to control the adjustment device.

Furthermore, the invention provides a method of treating morbid obesity in a human comprising: (a) Surgically implanting (preferably in a laparascopic surgery) in the human an elongated restriction member 12 defining a substantially closed loop (see FIGURE 2) around the human's stomach 14 or esophagus 15, defining a restriction opening 16. (b) Surgically implanting in the human an adjustment device which adjusts the restriction opening 16, and a pressure sensor 23 for sensing the pressure in the humans' stomach 14. (c) In response to sensing by the pressure sensor 23 of a pressure in the human's stomach 14 greater than a predetermined amount, controlling the adjustment device to reduce the size of the opening 16. And (d) in response to sensing by the pressure sensor 23 of a pressure less than a predetermined amount in the human stomach 14, controlling the adjustment device to substantially fully open the opening 16. The method may also comprise (e) controlling the adjustment device in response to the time of day (*e*.*g*. using a clock mechanism as described above, *e*.*g*. as part of the control unit 22) to vary the restriction opening 16 unless overridden by the pressure sensor 23 sensing pressure in the stomach 14 that would cause the stoma opening to be less than fully open, or "closed" (that is having a minimize size substantially preventing further passage of food particles into the stomach, or a part thereof).

FIGURE 3 shows an embodiment similar to that of FIGURE 2 with comparable components shown by the same reference numbers only preceded by a "1". In the FIGURE 3 embodiment, however, two chambers 26,27 are separated from each other in a fluid tight manner by a partition wall 28, and the pump 120 pumps fluid from one chamber 26 to the other chamber 27 to change the size of the restriction opening 116. The sensor 123 may in this case be a conventional position sensor, which senses the orientation of the patient with respect to the horizontal. Both the pump 120 and sensor 123 are fixed to the partition wall 28 inside the chambers 26,27. Conventional locking members 30 may be used to hold the elongated restriction member 112 in the formed loop. The control unit 122 is implanted remote from the restriction member 112 and operably connected to the sensor 123 and pump 120 through a line 31. As in the other embodiments the control unit 122 may include an internal clock mechanism, and may be controlled from externally of the human by wireless remote control (like the remote control 24 in FIGURE 2). If desired a pressure sensor 23 may also be included.

Alternative or additional sensors which sense other physical parameters related to sleep or rest of the human in which the sensors are implanted may also or alternatively be utilized.

While the invention has been herein shown and described in a preferred practical embodiment for the treatment of obese humans, it is to be understood by those of ordinary skill in the art that also patients suffering from heartburn and reflux disease can be treated with the apparatus 10 described. The scope of the invention is defined by the appended claims.

## Claims

1. An apparatus (10) for forming a stoma opening in the stomach (14) or esophagus (15) of a patient, the apparatus (10) comprising:
an implantable elongated restriction member (12) adapted to be formed into a substantially closed loop around the patient's stomach (14) or esophagus (15), so that the loop defines a restriction opening (16),
an implantable adjustment device (18) for adjusting the restriction member (12) in the loop to change the size of the restriction opening (16),
a control device (12) for controlling the adjustment device to adjust the restriction member (12) to change the size of the restriction opening (16), and
an implantable sensor (23) for sensing at least one physical parameter associated with the patient, **characterised in that** the control device is adapted to control the adjustment device in response to the sensor (23) sensing a change in the physical parameter.

2. An apparatus (10) according to claim 1, wherein the control device comprises an implantable internal control unit (22) for directly controlling the adjustment device in response to signals from the sensor (23).

3. An apparatus (10) according to claim 1, wherein the control device comprises an external control unit outside the patient's body for controlling the adjustment device in response to signals from the sensor (23).

4. An apparatus (10) according to claim 3, wherein the external control unit is adapted to directly control the adjustment device in response to signals from the sensor (23).

5. An apparatus (10) according to claim 4, wherein the external control unit is adapted to store information on the physical parameter sensed by the sensor (23) and to be manually operated to control the adjustment device based on the stored information.

6. An apparatus (10) according to any of claims 1 to 5, further comprising at least one implantable sender for sending information on the physical parameter sensed by the sensor (23).

7. An apparatus (10) according to any of claims 1 to 6, wherein the sensor (23) comprises a pressure sensor for sensing as the physical parameter the pressure in the patient's stomach (14) or esophagus (15).

8. An apparatus (10) according to claim 7, wherein the pressure sensor is adapted to indirectly sense the pressure in the stomach (14) by sensing the pressure exerted by the stomach (14) against the restriction member (12).

9. An apparatus (10) according to claim 7 or 8, wherein the control device is adapted to control the adjustment device to change the size of the restriction opening (16) in response to the pressure sensor sensing a change in the pressure in the stomach (14) or esophagus (15).

10. An apparatus (10) according to claim 9, wherein the apparatus (10) is intended for restricting the food intake of an obese patient, the control device being adapted to control the adjustment device to reduce the restriction opening (16) in response to the pressure sensor sensing a pressure equal to or exceeding a predetermined value.

11. An apparatus (10) according to claim 9 or 10, wherein the apparatus (10) is intended for restricting the food intake of an obese patient, the control device being adapted to control the adjustment device to enlarge the restriction opening (16) in response to the pressure sensor sensing a pressure below a predetermined value.

12. An apparatus (10) according to any of claims 9 to 11, wherein the apparatus (10) is intended for restricting the food intake of an obese patient, the control device being adapted to control the adjustment device to enlarge the restriction opening (16) in response to the pressure sensor sensing a pressure equal to or exceeding an excessively high value.

13. An apparatus (10) according to claim 9, wherein the apparatus (10) is intended for treating heartburn and reflux disease, the control device being adapted to control the adjustment device to enlarge the restriction opening (16) in response to the pressure sensor sensing a pressure equal to or exceeding a predetermined value.

14. An apparatus (10) according to claim 9 or 13, wherein the apparatus (10) is intended for treating heartburn and reflux disease, the control device being adapted to control the adjustment device to reduce or close the restriction opening (16) in response to the pressure sensor sensing a pressure below a predetermined value.

15. An apparatus (10) according to any of claims 10, 14 and 15, wherein the apparatus (10) is intended for treating heartburn and reflux disease, the control device being adapted to control the adjustment device to enlarge or fully open the restriction opening (16) in response to the pressure sensor sensing a pressure equal to or exceeding an excessively high value, to avoid injurious high pressures in the stomach (14) or esophagus (15).

16. An apparatus (10) according to any of claims 1 to 6, wherein the sensor (23) comprises a position sensor for sensing as the physical parameter the orientation of the patient with respect to the horizontal.

17. An apparatus (10) according to any of claims 7 to 15, further comprising a position sensor for sensing the orientation of the patient with respect to the horizontal.

18. An apparatus (10) according to claim 16 or 17, wherein the apparatus (10) is intended for restricting the food intake of an obese patient, the control device being adapted to control the adjustment device to increase the restriction opening (16) in response to the position sensor sensing that the patient has assumed a substantially horizontal orientation.

19. An apparatus (10) according to claim 16 or 17, wherein the apparatus (10) is intended for treating heartburn and reflux disease, the control device being adapted to control the adjustment device to close the restriction opening (16) in response to the position sensor sensing that the patient has assumed a substantially horizontal orientation.

20. An apparatus (10) according to claim 1, wherein the control device is adapted to control the adjustment device in response to the time of the day.

21. An apparatus (10) according to claim 20, wherein the control device comprises a clock mechanism used for controlling the adjustment device to adjust the restriction member to keep the restriction opening (16) at different sizes during different time periods of the day.

22. An apparatus (10) according to any of claims 1 to 6, wherein the control device comprises a clock mechanism used for controlling the adjustment device to adjust the restriction member to keep the restriction opening (16) at different sizes during different time periods of the day, provided that the physical parameter sensed by the sensor does not override the clock mechanism.

23. An apparatus (10) according to any of claims 1 to 22, wherein the control device comprises an implantable internal control unit (22), and further comprising a wireless remote control (24) adapted to set control parameters of the internal control unit (22) from outside the patient without mechanically penetrating the patient, when the internal control unit (22) is implanted in the patient.

24. An apparatus (10) according to claim 1 and 23, wherein at least one of the control parameters, which is settable by the wireless remote control (24), is the physical parameter.

25. An apparatus according to claim 23 or 24, wherein the internal control unit (22) includes a clock mechanism used for controlling the adjustment device to adjust the restriction member to keep the restriction opening (16) at different sizes during different time periods of the day.

26. An apparatus (10) according to claim 25, wherein the wireless remote control (24) is adapted to set the clock mechanism.

27. An apparatus (10) according to claim 1, wherein the control device comprises a wireless remote control (24).

28. An apparatus (10) according to claim 27, wherein the wireless remote control (22) comprises a signal transmitter, an implantable signal receiver, and an implantable energizer unit for transforming wireless energy from the signals, as they are transmitted from the signal transmitter to the signal receiver, into energy different than the wireless energy for energizing the adjustment device.

29. An apparatus (10) according to claims 1 and 28, wherein the apparatus is adapted to use the energy transformed from the wireless energy for energizing the implantable sensor (23).

30. An apparatus (10) according to claim 1, wherein the adjustment device comprises an expandable cavity (19) in the restriction member (12), the size of the restriction opening (16) being reduced upon expansion of the cavity (19) and increased upon contraction of the cavity (19), and a reservoir (21) for hydraulic fluid, the adjustment device being adapted to distribute hydraulic fluid from the reservoir (21) to expand the cavity (19), and to distribute hydraulic fluid from the cavity (19) to the reservoir (21) to contract the cavity (19), to thereby change the size of the restriction opening (16).

31. An apparatus (10) according to claim 30, wherein the reservoir (21) is attached to the restriction member (12).

32. An apparatus (10) according to claim 30, wherein the reservoir (21) is fixed to the restriction member (12).

33. An apparatus (10) according to claim 30, wherein the reservoir (21) is integrated with the restriction member (12).

34. An apparatus (10) according to any of claims 30 to 33, wherein the adjustment device comprises a pump (20) for pumping fluid between the cavity (19) and the reservoir (21).

35. An apparatus (10) according to claim 34, wherein the pump (20) is attached to the restriction member (12).

36. An apparatus (10) according to claim 34, wherein the pump (20) is fixed to the restriction member (12).

37. An apparatus (10) according to claim 34, wherein the reservoir (19), pump (20) and restriction member (21) form a single piece.

38. An apparatus (10) according to claim 34, wherein the reservoir (19), pump (20), sensor (23) and restriction member (12) form a single piece.

39. An apparatus (10) according to claim 34, wherein the pump (20) is adapted to be subcutaneously implanted in the patient remote from the restriction member (12).

40. An apparatus (10) according to any of claims 1 to 29, wherein the adjustment device is adapted to mechanically adjust the restriction member (12).

41. An apparatus (10) according to any of claims 1 to 40, further comprising an implantable battery for energizing the adjustment device.

42. An apparatus (10) according to any of claims 1 to 40, further comprising an implantable accumulator for energizing the adjustment device.

43. An apparatus (10) according to claim 42, wherein the accumulator comprises a capacitor.

44. An apparatus (10) according to any of claims 1 to 19, 24 and 29, further comprising an implantable battery for energizing the sensor (23).

45. An apparatus (10) according to any of claims 1 to 19, 24 and 29, further comprising an implantable accumulator for energizing the sensor (23).

46. An apparatus (10) according to claim 45, wherein the accumulator comprises a capacitor.

## Patentansprüche

1. Vorrichtung (10) zum Bilden einer Stomaöffnung in dem Magen (14) oder in der Speiseröhre (15) eines Patienten, wobei die Vorrichtung (10) aufweist:
ein implantierbares, längliches Restriktionselement (12), das dazu ausgelegt ist, in einer im Wesentlichen geschlossene Schleife um den Magen (14) oder die Speiseröhre (15) des Patienten geformt zu werden, so dass die Schleife eine Restriktionsöffnung (16) definiert,
eine implantierbare Einstellvorrichtung (18) zum Einstellen des in der Schleife vorliegenden Restriktionselementes (12), um die Größe der Restriktionsöffnung (16) zu ändern,
eine Steuervorrichtung (12) zum Steuern der Einstellvorrichtung, um das Restriktionselement (12) anzupassen, so dass die Größe der Restriktionsöffnung (16) geändert wird, und
einen implantierbaren Sensor (23) zum Abfühlen wenigstens eines physikalischen Parameters, der dem Patienten zugeordnet ist, **dadurch gekennzeichnet, dass** die Steuervorrichtung so ausgelegt ist, dass sie die Einstellvorrichtung als Antwort darauf, dass der Sensor (23) eine Änderung bei dem physikalischen Parameter erfasst, steuert.

2. Vorrichtung (10) nach Anspruch 1, bei der die Steuervorrichtung eine implantierbare interne Steuereinheit (22) zum direkten Steuern der Einstellvorrichtung als Antwort auf Signale von dem Sensor (23) aufweist.

3. Vorrichtung (10) nach Anspruch 1, bei der die Steuervorrichtung eine externe Steuereinheit außerhalb des Körpers des Patienten zum Steuern der Einstellvorrichtung als Antwort auf Signale von dem Sensor (23) aufweist.

4. Vorrichtung (10) nach Anspruch 3, bei der die externe Steuereinheit dazu ausgelegt ist, die Einstellvorrichtung direkt als Antwort auf Signale von dem Sensor (23) zu steuern.

5. Vorrichtung (10) nach Anspruch 4, bei der die externe Steuereinheit dazu ausgelegt ist, Information über den physikalischen Parameter, der von dem Sensor (23) abgefühlt wird, zu speichern, und von Hand bedient zu werden, um die Einstellvorrichtung basierend auf der gespeicherten Information zu steuern.

6. Vorrichtung (10) nach einem der Ansprüche 1 bis 5, die weiter wenigstens einen implantierbaren Sender zum Senden von Information über den physikalischen Parameter, der von dem Sensor (23) abgefühlt wird, aufweist.

7. Vorrichtung (10) nach einem der Ansprüche 1 bis 6, bei der der Sensor (23) einen Drucksensor zum Abfühlen des Druckes in dem Magen (14) oder in der Speiseröhre (15) des Patienten als den physikalischen Parameter aufweist.

8. Vorrichtung (10) nach Anspruch 7, bei der der Drucksensor dazu ausgelegt ist, den Druck in dem Magen (14) indirekt abzufühlen, indem der Druck abgefühlt wird, der von dem Magen (14) gegen das Restriktionselement (12) ausgeübt wird.

9. Vorrichtung (10) nach Anspruch 7 oder 8, bei der die Steuervorrichtung dazu ausgelegt ist, die Einstellvorrichtung so zu steuern, dass sie die Größe der Restriktionsöffnung (16) als Antwort darauf, dass der Drucksensor eine Änderung in dem Druck in dem Magen (14) oder in der Speiseröhre (15) erfasst, ändert.

10. Vorrichtung (10) nach Anspruch 9, wobei die Vorrichtung (10) zum Einschränken der Nahrungsaufnahme eines fettleibigen Patienten gedacht ist, wobei die Steuervorrichtung dazu ausgelegt ist, die Einstellvorrichtung so zu steuern, dass sie die Restriktionsöffnung (16) als Antwort darauf, dass der Drucksensor einen Druck abfühlt, der gleich einem vorbestimmten Wert ist oder ihn überschreitet, verkleinert.

11. Vorrichtung (10) nach Anspruch 9 oder 10, wobei die Vorrichtung (10) für das Einschränken der Nahrungsaufnahme eines fettleibigen Patienten gedacht ist, wobei die Steuervorrichtung dazu ausgelegt ist, die Einstellvorrichtung so zu steuern, dass sie die Restriktionsöffnung (16) als Antwort darauf, dass der Drucksensor einen Druck unterhalb eines vorbestimmten Werts abfühlt, vergrößert.

12. Vorrichtung (10) nach einem der Ansprüche 9 bis 11, wobei die Vorrichtung (10) für die Einschränkung der Nahrungsaufnahme eines fettleibigen Patienten gedacht ist, wobei die Steuereinrichtung dazu ausgelegt ist, die Einstellvorrichtung so zu steuern, dass sie die Restriktionsöffnung (16) in Antwort darauf, dass der Drucksensor einen Druck abfühlt, der gleich einem außerordentlich hohen Wert ist oder diesen übersteigt, vergrößert.

13. Vorrichtung (10) nach Anspruch 9, wobei die Vorrichtung (10) zum Behandeln von Sodbrennen und Refluxleiden gedacht ist, wobei die Steuervorrichtung dazu ausgelegt ist, die Einstellvorrichtung so zu steuern, dass sie die Restriktionsöffnung (16) als Antwort darauf, dass der Drucksensor einen Druck abfühlt, der gleich einem vorbestimmten Wert ist oder ihn überschreitet, vergrößert.

14. Vorrichtung (10) nach Anspruch 9 oder 13, wobei die Vorrichtung (10) zum Behandeln von Sodbrennen und Refluxleiden gedacht ist, wobei die Steuervorrichtung dazu ausgelegt ist, die Einstellvorrichtung so zu steuern, dass sie die Restriktionsöffnung (16) als Antwort darauf, dass der Drucksensor einen Druck unterhalb eines vorbestimmten Werts abfühlt, verkleinert oder verschließt.

15. Vorrichtung (10) nach einem der Ansprüche 10, 14 und 15, wobei die Vorrichtung (10) für die Behandlung von Sodbrennen und Refluxleiden gedacht ist, wobei die Steuervorrichtung dazu ausgelegt ist, die Einstellvorrichtung so zu steuern, dass sie die Restriktionsöffnung (16) als Antwort darauf, dass der Drucksensor einen Druck abfühlt, der gleich einem übermäßig hohen Wert ist oder diesen überschreitet, vergrößert oder vollständig öffnet, um schädliche hohe Drücke in dem Magen (14) oder in der Speiseröhre (15) zu vermeiden.

16. Vorrichtung (10) nach einem der Ansprüche 1 bis 6, bei der der Sensor (23) einen Positionssensor zum Abfühlen der Ausrichtung des Patienten in Bezug auf die Horizontale als den physikalischen Parameter aufweist.

17. Vorrichtung (10) nach einem der Ansprüche 7 bis 15, die weiterhin einen Positionssensor zum Abfühlen der Ausrichtung des Patienten in Bezug auf die Horizontale aufweist.

18. Vorrichtung (10) nach Anspruch 16 oder 17, wobei die Vorrichtung (10) zum Einschränken der Nahrungsaufnahme bei einem fettleibigen Patienten gedacht ist, wobei die Steuereinrichtung dazu ausgelegt ist, die Einstellvorrichtung so zu steuern, dass sie die Restriktionsöffnung (16) als Antwort darauf, dass der Positionssensor abfühlt, dass der Patient eine im Wesentlichen horizontale Ausrichtung angenommen hat, vergrößert.

19. Vorrichtung (10) nach Anspruch 16 oder 17, wobei die Vorrichtung (10) zur Behandlung von Sodbrennen und Refluxleiden gedacht ist, wobei die Steuervorrichtung dazu ausgelegt ist, die Einstellvorrichtung so zu steuern, dass sie die Restriktionsöffnung (16) als Antwort darauf, dass der Positionssensor abfühlt, dass der Patient eine im Wesentlichen horizontale Ausrichtung einnimmt, verschließt.

20. Vorrichtung (10) nach Anspruch 1, bei der die Steuervorrichtung dazu ausgelegt ist, die Einstellvorrichtung als Antwort auf die Tageszeit zu steuern.

21. Vorrichtung (10) nach Anspruch 20, bei der die Steuervorrichtung einen Uhrmechanismus aufweist, der zum Steuern der Einstellvorrichtung verwendet wird, um das Restriktionselement einzustellen, damit die Restriktionsöffnung (16) während verschiedener Zeitintervalle des Tages auf unterschiedlichen Größen gehalten wird.

22. Vorrichtung (10) nach einem der Ansprüche 1 bis 6, bei der die Steuervorrichtung einen Uhrmechanismus aufweist, der zum Steuern der Einstellvorrichtung verwendet wird, um das Restriktionselement einzustellen, damit die Restriktionsöffnung (16) während verschiedener Zeitintervalle des Tages auf unterschiedlichen Größen gehalten wird, vorausgesetzt, dass der physikalische Parameter, der von dem Sensor abgefühlt wird, den Uhrmechanismus nicht außer Kraft setzt.

23. Vorrichtung (10) nach einem der Ansprüche 1 bis 22, bei der die Steuervorrichtung eine implantierbare interne Steuereinheit (22) aufweist, und sie weiterhin eine drahtlose Fernsteuerung (24) aufweist, die dazu ausgelegt ist, Steuerparameter der internen Steuereinheit (22) von außerhalb des Patienten einzustellen, ohne mechanisch in den Patienten einzudringen, wenn die interne Steuereinheit (22) in den Patienten implantiert ist.

24. Vorrichtung (10) nach Anspruch 1 und 23, bei der wenigstens einer der Steuerparameter, der durch die drahtlose Fernsteuerung (24) einzustellen ist, der physikalische Parameter ist.

25. Vorrichtung nach Anspruch 23 oder 24, bei der die interne Steuereinheit (22) einen Uhrmechanismus umfasst, der zum Steuern der Einstellvorrichtung verwendet wird, um das Restriktionselement einzustellen, damit die Restriktionsöffnung (124) während verschiedener Zeitintervalle des Tages auf unterschiedlichen Größen gehalten wird.

26. Vorrichtung (10) nach Anspruch 25, bei der die drahtlose Fernsteuerung (24) dazu ausgelegt ist, den Uhrmechanismus einzustellen.

27. Vorrichtung (10) nach Anspruch 1, bei der die Steuervorrichtung eine drahtlose Fernsteuerung (24) aufweist.

28. Vorrichtung (10) nach Anspruch 27, bei der die drahtlose Fernsteuerung (22) einen Signalsender, einen implantierbaren Signalempfänger und eine implantierbare Anregungseinheit zum Umwandeln von drahtloser Energie aus den Signalen, während sie von dem Signalsender an den Signalempfänger gesendet werden, in Energie, die unterschiedlich von der drahtlosen Energie zum Betreiben der Einstellvorrichtung ist, aufweist.

29. Vorrichtung (10) nach den Ansprüchen 1 und 28, wobei die Vorrichtung dazu ausgelegt ist, die Energie, die aus der drahtlosen Energie umgewandelt worden ist, zum Betreiben des implantierbaren Sensors (23) zu verwenden.

30. Vorrichtung (10) nach Anspruch 1, bei der die Einstellvorrichtung einen aufweitbaren Hohlraum (19) in dem Restriktionselement (12), wobei die Größe der Restriktionsöffnung (16) beim Aufweiten des Hohlraums (19) verringert und beim Zusammenziehen des Hohlraums (19) vergrößert wird, und einen Behälter (21) für hydraulisches Fluid aufweist, wobei die Einstellvorrichtung dazu ausgelegt ist, hydraulisches Fluid aus dem Behälter (21) auszutragen, um den Hohlraum (19) aufzuweiten, und hydraulisches Fluid von dem Hohlraum (19) in den Behälter (21) zu transportieren, um den Hohlraum (19) zusammenzuziehen, um somit die Größe der Restriktionsöffnung (16) zu ändern.

31. Vorrichtung (10) nach Anspruch 30, bei der der Behälter (21) an dem Restriktionselement (12) befestigt ist.

32. Vorrichtung (10) nach Anspruch 30, bei der der Behälter (21) an dem Restriktionselement (12) festgelegt ist.

33. Vorrichtung (10) nach Anspruch 30, bei der der Behälter (21) einstückig mit dem Restriktionselement (12) ausgebildet ist.

34. Vorrichtung (10) nach einem der Ansprüche 30 bis 33, bei der die Einstellvorrichtung eine Pumpe (20) zum Pumpen von Fluid zwischen dem Hohlraum (19) und dem Behälter (21) aufweist.

35. Vorrichtung (10) nach Anspruch 34, bei der die Pumpe (20) an dem Restriktionselement (12) befestigt ist.

36. Vorrichtung (10) nach Anspruch 34, bei der die Pumpe (20) an dem Restriktionselement (12) festgelegt ist.

37. Vorrichtung (10) nach Anspruch 34, bei der der Behälter (19), die Pumpe (20) und das Restriktionselement (12) ein einziges Teil bilden.

38. Vorrichtung (10) nach Anspruch 34, bei der der Behälter (19), die Pumpe (20), der Sensor (23) und das Restriktionselement (12) ein einziges Teil bilden.

39. Vorrichtung (10) nach Anspruch 34, bei der die Pumpe (20) dazu ausgelegt ist, in den Patienten subkutan entfernt von dem Restriktionselement (12) implantiert zu werden.

40. Vorrichtung (10) nach einem der Ansprüche 1 bis 29, bei der die Einstellvorrichtung dazu ausgelegt ist, das Restriktionselement (12) mechanisch anzupassen.

41. Vorrichtung (10) nach einem der Ansprüche 1 bis 40, die weiter eine implantierbare Batterie zum Betreiben der Einstellvorrichtung aufweist.

42. Vorrichtung (10) nach einem der Ansprüche 1 bis 40, die weiterhin einen implantierbaren Akkumulator zum Betreiben der Einstellvorrichtung aufweist.

43. Vorrichtung (10) nach Anspruch 42, bei der der Akkumulator einen Kondensator aufweist.

44. Vorrichtung (10) nach einem der Ansprüche 1 bis 19, 24 und 29, die weiterhin eine implantierbare Batterie zum Betreiben des Sensors (23) aufweist.

45. Vorrichtung (10) nach einem der Ansprüche 1 bis 19, 24 und 29, die weiterhin einen implantierbaren Akkumulator zum Betreiben des Sensors (23) aufweist.

46. Vorrichtung (10) nach Anspruch 45, bei der der Akkumulator einen Kondensator aufweist.

## Revendications

1. Appareil (10) pour former une ouverture de stoma dans l'estomac (14) ou l'oesophage (15) d'un patient, l'appareil (10) comprenant :
→ un élément de restriction allongé implantable (12), adapté pour être formé en une boucle sensiblement fermée autour de l'estomac (14) ou de l'oesophage (15) du patient, de sorte que la boucle définisse une ouverture de restriction (16),
→ un dispositif de réglage implantable (18) pour ajuster l'élément de restriction (12) dans la boucle, afin de changer la taille de l'ouverture de restriction (16),
→ un dispositif de contrôle (12) pour contrôler le dispositif de réglage, afin de régler l'élément de restriction (12) pour changer la taille de l'ouverture de restriction (16), et
→ un capteur implantable (23) pour détecter au moins un paramètre physique associé au patient, **caractérisé en ce que** le dispositif de contrôle est adapté pour contrôler le dispositif de réglage en réponse au capteur (23) détectant un changement du paramètre physique.

2. Appareil (10) selon la revendication 1, dans lequel le dispositif de contrôle comprend une unité de contrôle interne implantable (22) pour contrôler directement le dispositif de réglage, en réponse à des signaux provenant du capteur (23).

3. Appareil (10) selon la revendication 1, dans lequel le dispositif de contrôle comprend une unité de contrôle externe, située hors du corps du patient pour contrôler le dispositif de réglage en réponse aux signaux provenant du capteur (23).

4. Appareil (10) selon la revendication 3, dans lequel l'unité de contrôle externe est adaptée pour contrôler directement le dispositif de réglage en réponse aux signaux provenant du capteur (23).

5. Appareil (10) selon la revendication 4, dans lequel l'unité de contrôle externe est adaptée pour stocker les informations sur les paramètres physiques détectés par le capteur (23) et pour être manuellement actionnée afin de contrôler le dispositif de réglage sur la base des informations stockées.

6. Appareil (10) selon l'une quelconque des revendications 1 à 5, comprenant en outre au moins un émetteur implantable pour envoyer les informations sur les paramètres physiques détectés par le capteur (23).

7. Appareil (10) selon l'une quelconque des revendications 1 à 6, dans lequel le capteur (23) comprend un capteur de pression pour détecter, comme paramètre physique, la pression dans l'estomac (14) ou l'oesophage (15) du patient.

8. Appareil (10) selon la revendication 7, dans lequel le capteur de pression est adapté pour détecter indirectement la pression dans l'estomac (14) en détectant la pression exercée par l'estomac (14) contre l'élément de restriction (12).

9. Appareil (10) selon la revendication 7 ou 8, dans lequel le dispositif de contrôle est adapté pour contrôler le dispositif de réglage, pour changer la taille de l'ouverture de restriction (16), en réponse au capteur de pression détectant un changement de pression dans l'estomac (14) ou l'oesophage (15).

10. Appareil (10) selon la revendication 9, dans lequel l'appareil (10) est conçu pour limiter la prise alimentaire d'un patient obèse, le dispositif de contrôle étant adapté pour contrôler le dispositif de réglage, afin de réduire l'ouverture de restriction (16) en réponse au capteur de pression, détectant une pression égale à une valeur prédéterminée ou excédant celle-ci.

11. Appareil (10) selon la revendication 9 ou 10, dans lequel l'appareil (10) est conçu pour limiter la prise alimentaire d'un patient obèse, le dispositif de contrôle étant adapté pour contrôler le dispositif de réglage, afin d'élargir l'ouverture de restriction (16) en réponse au capteur de pression, détectant une pression inférieure à une valeur prédéterminée.

12. Appareil (10) selon l'une quelconque des revendications 9 à 11, dans lequel l'appareil (10) est conçu pour limiter la prise alimentaire d'un patient obèse, le dispositif de contrôle étant adapté pour contrôler le dispositif de réglage, afin d'élargir l'ouverture de restriction (16) en réponse au capteur de pression, détectant une pression égale à une valeur excessivement élevée ou excédant celle-ci.

13. Appareil (10) selon la revendication 9, dans lequel l'appareil (10) est conçu pour traiter les brûlures d'estomac et le reflux gastro-oesophagien, le dispositif de contrôle étant adapté pour contrôler le dispositif de réglage, afin d'élargir l'ouverture de restriction (16) en réponse au capteur de pression détectant une pression égale à une valeur prédéterminée ou excédant celle-ci.

14. Appareil (10) selon la revendication 9 ou 13,
dans lequel l'appareil (10) est conçu pour traiter les brûlures d'estomac et le reflux gastro-oesophagien, le dispositif de contrôle étant adapté pour contrôler le dispositif de réglage, afin de réduire ou de fermer l'ouverture de restriction (16), en réponse au capteur de pression, détectant une pression inférieure à une valeur prédéterminée.

15. Appareil (10) selon l'une quelconque des revendications 10, 14 et 15, dans lequel l'appareil (10) est destiné à traiter les brûlures d'estomac et le reflux gastro-oesophagien, le dispositif de contrôle étant adapté pour contrôler le dispositif de réglage, afin d'élargir ou d'ouvrir totalement l'ouverture de restriction (16) en réponse au capteur de pression, détectant une pression égale à une valeur excessivement élevée ou excédant celle-ci, afin d'éviter des pressions élevées nocives dans l'estomac (14) ou l'oesophage (15).

16. Appareil (10) selon l'une quelconque des revendications 1 à 6, dans lequel le capteur (23) comprend un capteur de position pour détecter, comme paramètre physique, l'orientation du patient relativement à l'horizontale.

17. Appareil (10) selon l'une quelconque des revendications 7 à 15, comprenant en outre un capteur de position pour détecter l'orientation du patient par rapport à l'horizontale.

18. Appareil (10) selon la revendication 16 ou 17, dans lequel l'appareil (10) est conçu pour limiter la prise alimentaire d'un patient obèse, le dispositif de contrôle étant adapté pour contrôler le dispositif de réglage pour augmenter l'ouverture de restriction (16) en réponse au capteur de position, détectant le fait que le patient a pris une orientation sensiblement horizontale.

19. Appareil (10) selon la revendication 16 ou 17, dans lequel l'appareil (10) est conçu pour traiter les brûlures d'estomac et le reflux gastro-oesophagien, le dispositif de contrôle étant adapté pour contrôler le dispositif de réglage, afin de fermer l'ouverture de restriction (10) en réponse au capteur de position détectant le fait que le patient a pris une orientation sensiblement horizontale.

20. Appareil (10) selon la revendication 1, dans lequel le dispositif de contrôle est adapté pour contrôler le dispositif de réglage en réponse au moment de la journée.

21. Appareil (10) selon la revendication 20, dans lequel le dispositif de contrôle comprend un mécanisme d'horloge utilisé pour contrôler le dispositif de réglage, afin d'ajuster l'élément de restriction pour maintenir l'ouverture de restriction (16) à différentes tailles pendant différents moments de la journée.

22. Appareil (10) selon l'une quelconque des revendications 1 à 6, dans lequel le dispositif de contrôle comprend un mécanisme d'horloge utilisé pour contrôler le dispositif de réglage, afin d'ajuster l'élément de restriction pour maintenir l'ouverture de restriction (16) à différentes tailles pendant différents moments de la journée, à condition que le paramètre physique détecté par le capteur ne mette pas hors service le mécanisme d'horloge.

23. Appareil (10) selon l'une quelconque des revendications 1 à 22, dans lequel le dispositif de contrôle comprend une unité de commande interne implantable (22) et comprenant en outre une commande à distance sans fil (24) adaptée pour régler les paramètres de contrôle de l'unité de contrôle interne (22) provenant de l'extérieur du patient sans pénétrer mécaniquement dans le patient, quand l'unité de contrôle interne (22) est implantée dans le patient.

24. Appareil (10) selon la revendication 1 et 23, dans lequel au moins un élément parmi les paramètres de contrôle, qui est réglable par la commande à distance sans fil (24), est le paramètre physique.

25. Appareil selon la revendication 23 ou 24, dans lequel l'unité de contrôle interne (22) comprend un mécanisme d'horloge utilisé pour contrôler le dispositif de réglage, afin d'ajuster l'élément de restriction pour maintenir l'ouverture de restriction (16) à différentes tailles pendant différents moments de la journée.

26. Appareil (10) selon la revendication 25, dans lequel la commande à distance sans fil (24) est adaptée pour régler le mécanisme d'horloge.

27. Appareil (10) selon la revendication 1, dans lequel le dispositif de contrôle comprend une commande à distance sans fil (24).

28. Appareil (10) selon la revendication 27, dans lequel la commande à distance sans fil (22) comprend un émetteur de signal, un récepteur de signal implantable, et une unité d'activateur implantable pour transformer l'énergie sans fil provenant des signaux, quand ils sont transmis de l'émetteur du signal au récepteur de signal, en une énergie différente à l'énergie sans fil pour alimenter le dispositif de réglage.

29. Appareil (10) selon les revendications 1 et 28, dans lequel l'appareil est adapté pour utiliser l'énergie transformée de l'énergie sans fil pour alimenter le capteur implantable (23).

30. Appareil (10) selon la revendication 1, dans lequel le dispositif de réglage comprend une cavité extensible (19) dans l'élément de restriction (12), la taille de l'ouverture de restriction (16) étant réduite lors de la dilatation de la cavité (19) et augmentée lors de la contraction de la cavité (19), et un réservoir (21) pour un fluide hydraulique, le dispositif de réglage étant adapté pour distribuer du fluide hydraulique du réservoir (21) pour dilater la cavité (19), et pour distribuer le fluide hydraulique de la cavité (19) au réservoir (21) pour contracter la cavité (19), afin de changer ainsi la taille de l'ouverture de restriction (16).

31. Appareil (10) selon la revendication 30, dans lequel le réservoir (21) est fixé à l'élément de restriction (12).

32. Appareil (10) selon la revendication 30, dans lequel le réservoir (21) est fixé à l'élément de restriction (12).

33. Appareil (10) selon la revendication 30, dans lequel le réservoir (21) est intégré à l'élément de restriction (12).

34. Appareil (10) selon l'une quelconque des revendications 30 à 33, dans lequel le dispositif de réglage comprend une pompe (20) pour pomper du fluide entre la cavité (19) et le réservoir (21).

35. Appareil (10) selon la revendication 34, dans lequel la pompe (20) est attachée à l'élément de restriction (12).

36. Appareil (10) selon la revendication 34, dans lequel la pompe (20) est fixée à l'élément de restriction (12).

37. Appareil (10) selon la revendication 34, dans lequel le réservoir (19), la pompe (20) et l'élément de restriction (21) forment une seule pièce.

38. Appareil (10) selon la revendication 34, dans lequel le réservoir (19), la pompe (20), le capteur (23) et l'élément de restriction (12) forment une seule pièce.

39. Appareil (10) selon la revendication 34, dans lequel la pompe (20) est adaptée pour être implantée par voie sous-cutanée dans le patient loin de l'élément de restriction (12).

40. Appareil (10) selon l'une quelconque des revendications 1 à 29, dans lequel le dispositif de réglage est adapté pour ajuster mécaniquement l'élément de restriction (12).

41. Appareil (10) selon l'une quelconque des revendications 1 à 40, comprenant en outre une batterie implantable pour alimenter le dispositif de réglage.

42. Appareil (10) selon l'une quelconque des revendications 1 à 40, comprenant en outre un accumulateur implantable pour alimenter le dispositif de réglage.

43. Appareil (10) selon la revendication 42, dans lequel l'accumulateur comprend un condensateur.

44. Appareil (10) selon l'une quelconque des revendications 1 à 19, 24 et 29, comprenant en outre une batterie implantable pour alimenter le capteur (23).

45. Appareil (10) selon l'une quelconque des revendications 1 à 19, 24 et 29, comprenant en outre un accumulateur implantable pour alimenter le capteur (23).

46. Appareil (10) selon la revendication 45, dans lequel l'accumulateur comprend un condensateur.
